# EUROPEAN PATENT APPLICATION

(11) **EP 4 202 923 A1**
(43) Date of publication of application: **28.06.2023**
(21) Application number: 22212526.2
(22) Date of filing: 09.12.2022
(51) Int. Cl.: G10L 25/66, A61B 5/00

(54) **VOICE RECOGNITION BASED EVALUATION OF PARKINSON'S DISEASE CONDITION(S)**

(30) Priority: 22.12.2021 US 202163292570 P
(71) Applicant: Neuroderm, Ltd., 7670212 Rehovot (IL)
(72) Inventor: SHOR, Eran, 6248427 Tel Aviv (IL); BEN DAVID, Tamir, 6998827 Tel Aviv (IL); DAVID, Uri, Ness Ziona (IL)
(74) Representative: HGF

(57) **Abstract**

Systems and methods are provided for evaluating a Parkinson disease (PD) condition or a progression thereof by using voice analysis, and for treating the PD condition. Evaluation of the PD condition or of the progression thereof may be performed instantly or over time by analyzing current voice samples of the PD subject and, optionally, historical voice samples that are stored in a data storage unit, determining pertinent voice characteristics of the PD subject based on the analysis of the voice samples, and evaluating the PD condition, or the progression thereof by comparing current voice characteristics to similar voice characteristics. Treating a PD condition may include, for example, adjusting a drug delivery parameter to a level that is treatment-wise beneficial, for example, in preventing or delaying the onset of "off periods or shortening the duration of an "off period, or ameliorating a Hypokinetic dysarthria (HD) symptom or another PD symptom.

## Description

### FIELD OF THE INVENTION

The present invention generally relates to systems and methods for evaluating a Parkinson disease ("PD") condition of a subject from information extracted from the PD subject's voice (spoken words or phrases) and, in particular, by phonetically analyzing speech and other sound information caused by or originate from the PD subject. The present invention also discloses means for ameliorating PD symptoms based on the analyzed voice/speech and/or on the analysis of other sound information.

### BACKGROUND

Measurement of sound characteristics of certain body functions, particularly sounds related to speech, breathing (or cessation, or partial cessation, of breathing) and respiratory system provides important medical information that may be useful in diagnosing (and, in some cases, treating) Parkinson symptoms. For example, measurement of snoring intensity can be of importance. Other examples related to the respiratory system include intensity of wheezing (a shrill whistle or coarse rattle heard when the airway is partially blocked), stridor (a noisy or high-pitched sound with breathing that is heard when the upper airway is partially blocked), coughing, respiratory rates, etc., which may be clinically important in diagnosing various Parkinson conditions and other conditions. In addition, sounds produced by a subject's speech system are also clinically important in evaluating Parkinson conditions.

### Parkinson and voice/speech

In humans, four main body systems are involved in the production of speech. The respiratory system, laryngeal system and articulatory systems are responsible for the physical manifestations of speech, and the nervous system regulates these systems on both the conscious and unconscious levels.

Many people with Parkinson's disease (PD) experience problems with one or more of the four speech systems. The most common communication disorder associated with Parkinson is Hypokinetic dysarthria, which is a speech disorder that causes rigidity and slowness of the systems of communication including breathing, swallowing, voice and speech. Briefly, Hypokinetic dysarthria is characterized perceptually by varying degrees of reduced pitch variation (monotonicity), reduced loudness, breathy voice, imprecise consonants, variable (uncontrollable) speaking rate, and short rushes of speech. PD patient may experience one or more of the symptoms "reduced voice sound amplitude" (reduced vocal loudness), "reduced time of speech periods", "reduced fundamental frequency range", "hoarse voice quality", "monotone in conversation", "imprecise articulation", "consonant and vowel imprecision", "short rushes of speech", "irregular pauses", "vocal tremor" and "change in rate of speech". People with Parkinson might slur words, mumble, or trail off at the end of a sentence.

People listening to a PD subject suffering from speech problems may have increased difficulty in understanding the PD subject to the extent that the listeners may ask the PD subject to repeat what s/he said. Such communication problems can result in social isolation and loneliness of the PD subject, including difficulty in communicating the PD subject's needs to her/his health care providers and caregivers.

An article titled *"*Speech and language therapy treatment on Hypokinetic dysarthria in Parkinson disease: Systematic review and meta-analysis" (Natalia Munoz-Vigueras et al., first published on November 24, 2020) addresses an effort that was made to assess the effect of *Speech and Language Therapy* ("SLT") on Hypokinetic dysarthria (HD) in Parkinson disease, and the article suggests a beneficial effect of SLT for reducing HD in Parkinson disease subjects, improving perceptual intelligibility, sound pressure level and semitone standard deviation. SLT has been found to be effective if targeted to patients with specific speech disorder and needs and provided intensively.

PD subjects typically intake medications (e.g., levodopa) to relieve some of the PD symptoms (e.g., HD symptoms) that are caused by Parkinson disease. It would, therefore, be beneficial, on the one hand, to characterize (e.g., classify, rank or score severity of) HD symptoms, or other types of PD conditions/symptoms, and, on the other hand, to be able to use the medicament itself to treat or ameliorate PD symptoms (e.g., HD symptoms), improve speech intelligibility so that a PD subject's speech is comprehensible to a listener, etc., rather than overburdening the PD subject with additional type of therapy, for example SLT.

### SUMMARY OF THE INVENTION

Systems and methods are provided for evaluating a PD condition and a progression of a PD condition by analyzing voice data of the involved PD subject, and optionally other sounds that originate from (or caused by) the PD subject. Progression of a PD condition in a PD subject may be evaluated based on analysis of historical and current voice data, and other sound data, that are related to (originate from) the PD subject.

In some embodiments, evaluation of a PD condition may be used to prevent or minimize the number of onsets of "off" periods in PD subjects by, for example, evaluating speech clarity/intelligibility through analysis of voice/speech samples. For example, a system may determine (predict) a future onset and/or duration of an imminent "off" period based on speech evaluation. The system may train a machine learning algorithm to predict a future "off" period through correlation (association) between historical (previous) "off" periods and historical (previous) voice/speech characteristics. The system may output (e.g., by using the system's machine learning algorithm) a 'predictive value' that is a number that indicates the likelihood of the occurrence (onset and/or duration) of a future (e.g., imminent) "off" period. The system may use the predictive value to adjust a parameter of a medicament intake in a PD subject in a way that would prevent the occurrence of the expected (imminent) "on" period, or minimize the likelihood of its occurrence, or delay its occurrence as much as possible, or shorten the duration of the "off" period. Adjusting the parameter of the medicament intake may be performed by adjusting a suitable operational parameter of the medicament delivery device/system that delivers the medicament to the PD subject. (The medicament delivery device may be part of the system or a standalone device.) Adjustment of the operational parameter of the medicament delivery device may be performed manually (e.g., by the PD subject), or automatically (by the medicament delivery device itself).

In some embodiments, systems and methods may ameliorate Hypokinetic dysarthria (HD) symptoms (or other types of PD symptoms) in PD subjects by evaluating voice/speech clarity/intelligibility. For example, a HD severity value, which is a special case of a PD severity value, may be determined (e.g., calculated) by the system to evaluate the severity of the HD symptoms and other (non-HD) PD symptoms in a PD subject by analyzing voice/speech characteristics/features that the system may idntify in word(s), phrase(s) or sentence(s) that are spoken (vocally repeated) by the PD subject. A PD severity value is a number indicating a severity of a PD symptom. HD severity value (a special case of the PD severity value) is a number indicating the severity of speech impairment, for example the extent to which word(s)/phrase(s)/sentence(s) spoken by the PD subject are unclear (unintelligibile). The system may monitor the HD severity value (or, in general, the PD severity value) over time (e.g., during the day) to determine whether a HD symptom (or PD symptom/condition) is steady, improving or worsening. Based on the monitored HD severity value (or monitored PD severity value), the system may adjust a parameter of a medicament intake by a PD subject in a way that would alleviate the HD symptom (or PD symptom or condition). Adjusting the parameter of the medicament intake may be performed by adjusting a suitable operational parameter of the medicament delivery device that delivers the medicament to the PD subject. Adjustment of the operational parameter of the medicament delivery device may be performed manually (e.g., by the PD subject), or automatically (by the medicament delivery device itself).

After the system recognizes a distortion (abnormality) in a word/phrase/sentence that was spoken by the PD subject, the system may recognize the spoken word/phrase/sentence and reconstruct (correct) the distorted word/phrase/sentence and vocally output the reconstructed word/phrase/sentence. This system's feature may be useful, for example, in cases where the PD subject wants to verbally interact with other people, and/or with the medicament delivery device (e.g., transferring a vocal command to the medicament delivery device), and in general with any application that includes (uses) a voice-user interface (VUI).

HD severity (or PD severity in general) evaluation may be useful for evaluating HD symptoms as well as for evaluating severity of other PD symptoms/conditions. Evaluating severity of PD conditions can be done by, for example, evaluating HD parameters (e.g., speech clarity) and/or by evaluating other parameters that are related to speech but not necessarily to HD symptoms. For example, the length of a phone conversation, the number of words spoken per minute, the total minutes of phone calls per day, are voice parameters that are related to speech but not necessarily to HD symptoms.

### BRIEF DESCRIPTION OF THE DRAWINGS

Various exemplary embodiments and aspects are illustrated in the accompanying figures with the intent that these examples be not restrictive. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. Of the accompanying figures:
Fig. 1 schematically illustrates a system according to an example embodiment;
Fig. 2 schematically illustrates a system according to another example embodiment;
Fig. 3 shows a method for treating "off' periods in a PD subject according to an example embodiment; and
Fig. 4 shows a method for treating HD symptoms in a PD subject according to an example embodiment.

### DETAILED DESCRIPTION OF THE INVENTION

The description that follows provides various details of example concepts and embodiments. However, this description is not intended to limit the scope of the claims but instead to explain various principles of the invention and exemplary manners of practicing it.

In one embodiment the invention involves using a system for monitoring "on" and "off" periods of a PD subjec by using voice analysis/recognition. The system may include a voice (sound) sensor for measuring or sampling the patient voice. The voice sensor may be, for example, part of a smartphone ("SP"), or part of a voice assist ("VA") device (e.g., Alexa), or a dedicated voice sensor. (Alexa is Ama'on's voice-based AI-powered digital assistant that powers an entire smart device ecosystem. Alexa is put to work by giving it a command by an Echo device, which is a hands-free speaker controlled with voice that uses speech recognition to perform tasks or commands given by the user audibly. In some cases, voice sensors can be wearable on (worn by) the patient. In some cases, multiple sensors may be located in the patient premises (e.g., home) to cover as many locations as practically possible of the patient during day and/night night.

The system may include a storing and computation unit for storing voice data and for analyzing the stored voice data, and, in addition, for performing signal processing and/or training a machine learning mechanism by using historical voice data. The storing and computation unit may be part of, for example, a smartphone (SP), a SP application or a voice assist (VA) device. The storing and computation unit may be a dedicated unit.

The system may optionally include a user interface to enable the PD subject to mark "on" and "off' periods. The user interface may be, for example, part of a SP application, or part of a dedicated device. The marked "on" and "off" periods, which may be historical "on" and "off" periods, may be used in conjunction with stored voice data as training data to train a machine learning model. Historical voice data and historical "on" and "off" periods may be recorded every predefined time interval, for example once a day, once a week, once a month, or according to any other interval that may be set, for example, according to a patient preference.

The system may optionally include a drug pump control unit to activate a drug delivery devce (pump) based on the condition of the PD subject. In some cases the drug delivery device may be configured to operate according to historical (previously marked) "on" and "off" periods, or to minimize the "off" periods, or when an "off" period is detected.

The system may optionally use a dedicated voice protocol to tune, calibrate or train the system, and/or to enhance accuracy of the voice analysing process, or to determine if a drug dose administered at a particular time is optimal. The voice protocol specifies a list of preselected word(s)/phrase(s)/sentence(s) that the system may prompt a PD subject to say (for the system to analyze) in order to enable the system to evaluate the severity of a potential problem associated with the PD subject's impaired, or ill-functioning, speech system. The system may use the voice protocol as a reference (fiducial indicia) voice information against which the system compares word(s)/phrase(s)/sentence(s) that are spoken by PD subject. Using the voice protocol enables the system to evaluate severity of HD symptoms (and PD symptoms in general) in PD subjects. The preselected protocl words/phrases/sentences are selected according to their ability to identify one or more speech problems. For example, the protocol words/phrases/sentences may be selected indivdually or collectively in a way that would enable the system to identify one or more speech problems of the PD subject. While the system may successfully identify a particular speech problem of the PD subject when the subject says (speaks out) a single protocl word or phrase, it may, in other scenarios, require the PD subject to say more than one phrase in order to enable the system to identify other speech problems.

The system may audibly and/or textually output protocol word(s)/phrase(s)/sentence(s) and prompt a PD subject to vocally repeat them for comparative analysis; i.e., for comparing the PD subject's spoken word(s)/phrase(s)/sentence(s) to the genuine (normal, genuine, undistored) word(s)/phrase(s)/sentence(s). For example, in some cases the system may audibly provide to the PD subect a protocol word/phrase and prompt the patient to vocally repeat (speak out) the word/phrase verbatim while the system simultaneously records the spoken word/phrase for comparative analysis vis-à-vis the pre-recorded reference protocol word/phrase. Similarly, the system may output a word or phrase in the form of written/typed text, for exampel using a computer screen, and prompt the PD subject to read the word/phrase for comparative analysis vis-à-vis the pre-recorded reference protocol word/phrase(s). A protocol sentence may be, or include, a sequence of vocal sounds, where a vocal sound may include alphabetic letters, vowels and/or consonants.

Using protocol words/phrases/sentence(s), the system may recognize the spoken protocol word(s)/phrase(s)/sentence(s) and phonetically compare them to reference word(s)/phrase(s)/sentence(s) in order to evaluate the severity and/or worsening of a speech problem of the PD subj ect over time. In some cases, the system may use the recognized protocol spoken word(s)/phrase(s)/sentenc(s) to phonetically correct words/phrases/sentence(s) that the PD subject speak during the day. The system may use the protocol word(s)/phrase(s)/sentence(s) as a training speech to learn and model speech patterns of the PD subj ect.

The system may optionally activate a voice sampling procedure during 'regular' (random) phone calls or during phone calls that are set in advance to specific timing. If an onset of an "off' period is predicted (expected) to occur at a specific time, timing of a phone call may be set to occur during the expected period of "on" and/or "off".

The system may activate the voice sampling procedure during sleep and measure breathing sounds, snoring sounds, and noises caused by bed movements. The system may use other types of sensors, for example movement sensors, and use them, for example in combination with voice analysis, to optimize, or enhance, detection of a speech problem of a PD subject, or another PD condition of the PD subject.

In some embodiments, detection of a PD condition (for example detection of "on" and "off periods) by the system is performed by using one or more of the following techniques:
1. Using a machine learning algorithm to identify "on" and "off periods (e.g., "off period onset and duration) based on voice data related to historical "on" and "off periods that are used as a training set. For example, a deep neural network ("DNN") may be trained to distinguish between the two Parkinsn states/conditions "on" and "off". To train a DNN, data derived from and/or associated with a plurality of factors known a priory to be related to (e.g., caused by or during) the "on" and "off states/conditions of PD patients may be used to fine tune the DNN in a way that would enable the DNN to classify similar data as either "on" state or "off state;
2. Measuring (monitoring) changes in a voice parameter, for example changes in voice volume, amplitude and intonation, and monitorig speech clarity. Speech clarity can be evaluated, for example, by comparing a word/phrase/sentence as spoken by a PD subject to a 'normal' (expected clear, 'genuine') sound of the same word/phrase/sentence, or to the same word/phrase/sentence as previously spoken by the PD subject. The sound difference between the voice produced by a PD subject to the expected sound can quantify a level of speech clarity. For example, the smaller the difference between a word/phrase/sentence spoken by a PD subject and a related expected sound, the clearer the speech;
3. Calculating an overall score (e.g., an overall HD severity value) for a PD condition per, for example, hour, day, week or month, and
4. Using voice recognition/analysis for monitoring comorbid neurological conditions along with monitoring of the PD condition. Such neurological conditions may include, for example, depression, dementia, Alzheimer, etc.

### Depression

Depression involves mood disorders. Patients with mild depression show symptoms of depression, anxiety, loss of interest and low self-assessment; while patients with severe depression will be pessimistic, desperate, hallucinatory delusional, physically declined, and even suicidal. People with depression can be relieved and cured by medication, psychological and physical means.

Clinical observations and studies have found significant correlation between characteristics and the depression degrees. The language features of depression patients are being slow, monotonous, and overcast, which are different from normal population. Depression recognition technology based on speech signals has been the audio focus of scholars due to its advantages (e.g., low cost, easy collection of data, and 'non-contact' technology). For example, Automatic Speech Depression Detection (ASDD) aims to explore the speaker's inner emotions and psychological activities by analyzing their speech signal. In another example, an audio depression regression model may be used to evaluate depression. The audio depression regression model may use a multiscale audio differential normalization (MADN) feature extraction algorithm.

### Dementia

Impairment in cognition is a common manifestation among individuals suffering from dementia, of which Alzheimer's disease (AD) is the most common. Speaking is a complex cognitive skill, so analysis of voice recordings of PD subjects can indicate the cognitive state of the PD subjects. Deep learning techniques (e.g., long short-term memory (LSTM), convolutional neural network (CNN)) may be used to process voice recordings in an automated fashion to classify dementia status in PD subjects.

In some embodiments, a device is used to reconstruct PD patient voice outcome (i.e., improve speech quality) by:
- Automatically correcting distorted voice sound and/or low-level voice sound.
- Comparing good quality ('genuine') historical voice records (sound), as reference, to the PD subject's actual (e.g., impaired) voice, and using the comparison result for voice correction.
- Convert text massage into intelligible voice massage.

Reconstructing the PD patient voice outcome enables vocal interaction between the PD subject and a drug delivery pump filling station, a technical support, an Assist Device, a digital companion, etc.

In addition to determing progression of a PD subject condition, the present invention also involves determining a state (severity) of a PD condition in a PD subject. As described herein, historical personalized voice data may be used to enhance accuracy of "on" and "off" periods detection in a PD subject, and to enhance accuracy of PD condition detection. However, detection of "on" and "off' periods and PD conditions can be implemented without using historical voice data. For example, such detection may be based on voice parameters' instant (e.g., current) and/or changing values.

### Prediction of onset and duration of "off" periods

In some embodiment, the system uses voice recognition to calculate, based on the PD subject's own voice, a predictive value indicative of a time of a future (e.g., imminent, next) onset of an "off' period in the PD subject and, optionally, the duration of the future "off' period. The predictive value may be used in the following ways:
(1) The system may provide the predictive value (e.g., output it visually and/or audibly) to the PD subject (or to another user of the system, e.g., to a therapist/caregiver), and recommend to the PD subject (or to the caregiver) to adjust a parameter of her/his daily, or current, medication intake (e.g., dose quantity or flowrate of the medicament intake) to a recommended value so as to prevent, or minimize the probablity of, the occurrence of the future (e.g., imminent) onset of an "off' period. In response to the the system outputing (visually and/or audibly) the predictive value, the PD subject may adjust the parameter of the medicament intake according to the system's recommendation. Optionally, the system may recommend to the PD subject (or to the caregiver) to adjast the parameter to a recommended value without providing the predictive value to the PD subject.
(2) The system may, based on the calculated predictive value, automatically adjust the parameter of the medication intake, and the parameter adjustment process performed by the system may be either transparent to the user or not.

The system may predict a future/imminent "off' period from voice samples/recordings by determining charateristics of historical voice data that preceded historical "off" periods, and monitoring a change trend in the PD subject's voice over time to indentify similar characteristics in current voice data. Another way to predict a future/imminent "off" period from voice samples/recordings may also be based on historical (e.g., recorded/stored) voice data, but, in this case, the system may evaluate historical voice data at the same time of day and see whether the PD subject was in "off" state. This approach is based on the notion that daily "off" periods tend to occur at the same time(s), or at similar time(s), of the day. Alternatively, the system may monitor changes in the historical voice data, and determined whether the speech quality (speech intelligiblity)/clarity of the PD subject has improved or worsened over time. If the speech quality/clarity of the PD subject has worsened over time, this may mean that an onset of the next "off" period is expected (predicted) to occure earlier than the time of day when the history "off" period occured.

Adjusting a parameter of the medicamenbt intake - if the system predicts (based on voice) that an imminent "off" period will commence in, for example, 60 minuts, the system may increase a medicament delivery dose accordingly, taking into consideration the uptake time of the medicament. For example, if the medicament uptake time is, for example, 30 minutes, the system will increase the dose at least 30 minutes before the predicted time of the "off" period onset.

### Ameliorating HD symptoms

In some cases, the system uses voice/speech recognition to recognize spoken words/phrases/sentences of a PD subject. Then, based on the recognized spoken words/phrases/sentences, the system calculates a "HD severity value". The calculated "HD severity value" is a value that quantifies the extent to which the PD subject's spoken words, phrases or sentences are distorted, for example with respect to reference words, phrases or sentences, or with respect to the PD subject's previous spoken words, phrases or sentences. For example, a greater severity value may indicate or represent a greater distortion in the recognized PD subject's spoken word(s), phrase(s) or sentence(s).

The system may monitor the severity of HD symptom(s) (i.e., HD symptom(s) of interest) and evaluate the effectiveness of medicament intake on HD symptom(s) based on the monitored HD severity value(s). Monitoring the severity of HD symptom(s) means applying voice recognition to the PD subject's spoken word(s)/phrase(s)/sentence(s) repeatedly, continuously, intermittently, occassionaly or 'on demand' (by the PD subject using a user interface), and calculating a HD severity value after each application of the voice recognition.

Evaluating the effectiveness of medicament intake by the system may be performed by:
(i) calculating, by the system, a HD severity value, or a series of HD severity values, and adjusting the value of a parameter of the medicament intake according to the calculated HD severity value(s);
(ii) operating the medicament delivery device (a pump) to deliver medicament to the PD subject according to the adjusted value of the parameter, and
(iii) recalculating, by the system, a HD severity value (or a series of HD severity values) after delivery of the medicament to the PD subject, or during medicament delivery, and determining, based on the recalculated HD severity value (or based on the recalculatd series of HD severity values), whether there is alleviation of the treated/target HD symptom(s), or whether there is exacerbation of the treated/target HD symptom(s) (i.e., if there is an increase in the severity of symptom(s)).

In some embodiments, the medicament delivery device may receive an input signal/data that represent a 'predictive value' associated with the onset of "off' periods, or it may calculate the predictive value based on the PD subject's voice. The medicament delivery device may use the received input signal/data, or the calculated predictive value, to adjust a parameter of the medicament delivery so as to prevent, or delay, the occurrence of an imminent "off" period. In other instances the medicament delivery device may receive an input signal/data that represent a 'HD severity value' that is associated with HD symptom(s), or calculate the HD severity value based on analysis of the PD subject's voice. The medicament delivery device may use the HD severity value to adjust a parameter of the medicament medicament delivery so as to relieve the HD symptom(s).

In some embodiments, the system may relieve the HD symptom(s) a PD subject suffers from, so that the PD subject may be able to vocally interact with other people, and, in addition, with every device or system that is configured to respond to vocal commands. This way, the PD subject may be able to vocally interact with the medicament delivery device, and with any computer application that is configured for vocal interaction.

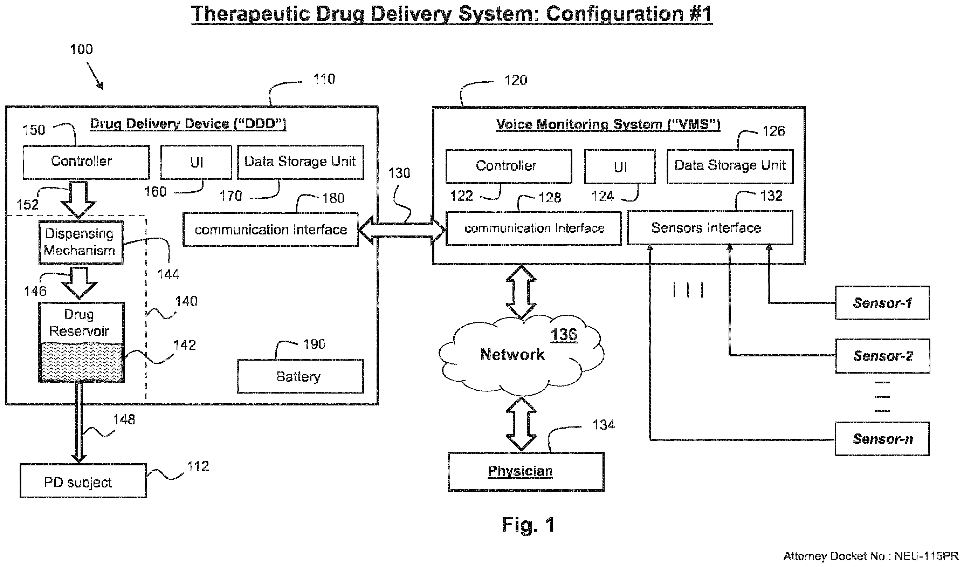

Fig. 1 shows a first configuration of a therapeutic drug delivery system (**100**) for treating a condition associated with a PD subject, for example for preventing or delaying the onset of future (e.g., imminent) "off" periods and/or minimizing the length of "off" periods, and for ameliorating Hypokinetic dysarthria (HD) symptoms/disorders in a PD subject. In this configuration, therapeutic drug delivery system **100** may include two, separate, subsystems: (1) a drug delivery device ("DDD") **110,** and (2) a voice monitoring system ("VMS") **120.** The two subsystems (**110,120**) may utilize a same communication protocol, and communicate with one another (e.g., exchange data and commands) via a suitable communication channel **130.** Communication channel **130** may be implemented by a computer communication cable (for example coaxial cable, Ethernet cable, USB cable, etc.), or wirelessly by using any known communication protocol (e.g., BlueTooth, WiFi, GSM (global system for mobile communications), etc.).

DDD **110** may include a controllable drug delivery unit ("DDU") **140,** a controller **150** for controlling (**152**) operation of DDU **140,** a user interface ("UI") **160,** a data storage unit ("DSU") **170,** a communication interface **180,** and a battery **190** (rechargeable or finite) for powering DDD **110.** Communication interface **180** can be configured to, for example, communicate with VMS **120** (as shown in Fig. 1) or, in various embodiments, with any data storage medium (e.g., the cloud or a memory of a server, or a computer hosted by a third-party tracker, such as, for example, a FitBit, Whoop Band, Apple Watch, etc.) DDD **110** may be designed to be wearable, or attachable to, a PD subject **112.** DDU **140** may include a therapeutic drug reservoir **142** for storing therein a therapeutic drug, and a controllable (**152**) dispensing mechanism **144** that is designed (mechanically or electrically, or both mechanically and electrically) with (it includes) a driving mechanism that is operative to expel (**146**) therapeutic drug out from therapeutic drug reservoir **142,** in controlled manner (**146**), so as to deliver the expelled therapeutic drug to patient **112,** for example via an infusion catheter **148.**

Controller **150** may be configured to receive voice data via communication interface **180** and/or to record spoken words by using user interface **160** that includes a voice/sound sensor (microphone). The voice data represent a word, a phrase, or a sentence that the PD subject says/speaks and recorded/stored, for example in DSU **126** or in DSU **170,** in order for them to be evaluated (for example by controller **150** or controller **122**), for example by comparing the word/phrase/sentence to fiducial word/phrase/sentence (e.g., to reference/protocol spoken word/phrase/sentence). Controller **150** (or controller **122**) may analyze the voice data vis-à-vis the reference spoken word/phrase/sentence, and, based on the voice analysis result the controller (**150** or **122**) may calculate a 'predictive value' to predict the onset of a future (e.g., imminent) "off" period. For example, the higher (greater) the value of the predictive value, the lesser the clarity of the word/phrase/sentence spoken by the PD subject (i.e., the more distorted the spoken word/phrase/sentence.), and, therefore, the shorter the time to the next onset of an "off" period. Depending on the predictive value (hence the estimated time to the next onset of an "off" period), controller **150** may adjust one or more operational parameters of controller **150** to adjust the operation of drug dispensing mechanism **144** in a way that would prevent the onset of the future "on" period or delay the onset of the future "off" period, and/or timewise shorten the length of the "off" period.

According to an aspect of the present invention the controller (**150** or **122**) may monitor the trend of the predictive values, for example by sampling the PD subject's voice over a period of time and calculating corresponding predictive values, and if the trend of the predictive values indicates that a next "off" period is due earlier than initially indicated (calculated or expected), the controller (**150** or **122**) may adjust the operational parameters of drug delivery unit (DDU) **140** in a way that DDU **140** would increase the amount of medicament that DDU **140** delivers to the PD subject. Controller 122 may be configured to monitor the PD subject's voice and calculate the predictive values, and it may also calculate the operational parameters for DDU **140.** Controller **150** may be configured to receive the predictive values and/or the values for the operational parameters of DDU **140** via communication interface **180.**

User interface (UI) **160** enables a user of DDD **110** (for example a physician, a PD patient or a caregiver helping a PD patient) to manually store various types of data (e.g., voice data representing word(s)/phrase(s)/sentence(s) spoken by a PD subject, data representing predictive values, data representing HD severity values) in DSU **170.** User interface (UI) **160** may also enable the user of DDD **110** to set therapeutically effective values of one or more operational parameters for controller **150.** The value(s) that the user may set to the operational parameters may also be stored in DSU **170.** Controller **150** may implement, or use, the parameters' values to control the operation of drug dispensing mechanism **144** according to these value(s).

The values which the user may set to the operational parameters of controller **150** may be selected such that applying them (e.g., by controller **150**) to drug dispensing mechanism **144** would prevent the next onset of an "off" period or delay its occurrence, or timewise shorten the "off" period. Example operational parameters that controller **150** may use to operate drug dispensing mechanism **144,** can be drug flow rate, drug delivery timing (including drug delivery start time and/or drug delivery stop time), increase of drug flow rate per unit of time, decrease of drug flow rate per unit of time, etc., or any combination thereof.

As explained herein, the respiratory system, laryngeal system and articulatory systems are responsible for the physical manifestations of speech, and, being a progressive disease, PD deteriorates voice clarity in PD patients over time, causing Hypokinetic dysarthria (HD) symptoms. According to an aspect of the present invention the controller (**150** or **122**) may monitor speech clarity of the PD subject, for example by sampling the PD subject's voice over a period of time, and calculate corresponding HD severity values, and if the trend of the HD severity values indicates that the PD subject's voice/speech is becoming unclear or deteriorates, the controller (**150** or **122**) adjusts the operational parameters of drug delivery unit (DDU) **140** in a way that DDU **140** would increase the amount of medicament that DDU **140** delivers to the PD subject, so as to ameliorate the HD symptom(s). Controller **122** may be configured to monitor the PD subject's voice and calculate the predictive values and/or the HD severity values, and the controller may also adjust the operational parameters for DDU **140** according to the predictive values and/or the HD severity values. Controller **150** may be configured to receive the predictive values and/or the HD severity values and/or the values for the operational parameters of DDU **140** via communication interface **180.** Depending on the time left until the onset of the next "off" period or on the type of HD symptom that is to be ameliorated, different (or additional) operational parameters of DDU **140** may be used.

User interface (UI) **160** may additionally output an informative feedback signal (visual, audible, and/or haptic) to the user with regard to the stored data and/or with regard to values which have been set by the user to the operational parameters, and/or with regard to the resulting instantaneous (current) drug delivery flow rate and/or drug delivery timing which controller **150** is currently implementing, or which controller **150** is scheduled to implement, and/or with regard to a relevant predictive value and the associated time of the next onset of an "off" period, and/or with regard to a relevant HD severity value and the associated speech clarity.

As described herein (for example above), the user of DDD **110** may use UI **160** to set therapeutically effective value(s) to the operational parameters of controller **150** to enable controller 150 to control the operation of drug dispensing mechanism **144** according to these value(s). Alternatively, controller **150** may use communication interface **180** to receive voice data that is based on, or derived from, signals that originate from one or more voice sensors. A voice sensor may be wearable by, or otherwise connected to, the treated PD subject. Controller **150** may use any known algorithm to analyze the voice data it receives via communication interface **180.** Controller **150** may determine (e.g., calculate) the values of the operational parameters of the DDU 140 from, or based on, the voice data. The voice data that controller **150** may receive via communication interface **180** and the parameters' values determined (e.g., calculated) by controller **150** may also be stored in DSU **170.** Controller **150** is configured to deliver a therapeutic drug composition (or compound) to the PD subject in a drug delivery pattern ("DDP") that is advantageous in treating condition, be it preventing or delaying onset of "off" periods (or timewise shortening "off" periods) or ameliorating HD-related speech unintelligibility. DDP refers to the drug flow rates at which drug is delivered to a PD subject during treatment, and to the timing and duration of each drug flow rate.

Voice monitoring system (VMS) **120** may include controller **122,** a user interface (UI) **124,** a data storage unit (DSU) **126,** a communication interface **128,** and a sensors' interface **132.** Communication interface **128** and communication interface 180 are configured (electrically and software wise) to communicate with one another via communication channel **130.**

Sensors interface **132** may be wired, or be wirelessly connected, to a number **n** of sensors (denoted "*Sensor-1*", *"Sensor-2"*,..., *"Sensor-n"* in Fig. 1). One or more of the ***n*** sensors may be sound/audio sensors to facilitate recording of voice/speech. Other sensors may be, for example, an electrode, a neuronal activity sensor, an EEG sensor, an ECG sensor, an EMG sensor, a polysomnography (PSG) sensor, a sleep sensor, a sleep state sensor, a local field potential sensor, an accelerometer, a movement sensor, a location sensor, a wristwatch configured to detect movements of a PD subjects during sleep, an optical sensor, a camera, etc. Some of the sensors may be capable of sensing spatial (3-D) movements of the PD subject.

In some embodiments VMS **120** is a standalone (i.e., functionally disconnected from DDD **110**) system configured to perform all the functions and tasks involving, for example, monitoring PD subject's voice, storing voice samples, analyzing/characterizing historical and current voice, calculating predictive values, calculating HD severity values (or other types of PD severity values), etc. For example, to monitor voice characteristics of PD subjects and determine a progression in a PD condition of the PD subject from the monitored voice characteristics controller **122** of VMS **120** may operate in a similar way as controller **150** of DDD **110.** In these embodiments VMS **120** may interact with a remote computer or server (for example with a physician's computer **134**) via a data network **136.** An example interaction between VMS **120** and physician computer/server **134** may include transferring voice data via network **136** that represent, or is related to, the PD subject's voice from VMS **120** to physician's computer **134.** In response to the voice data that physician computer/server **134** receives, physician computer/server **134** may return to VMS **120** (via network **136),** hence to the involved PD subject, instruction(s) or information regarding voice monitoring parameters. Physician computer/server **134** may also send to VMS **120** recommendations regarding optimization of the treatment of the PD condition vis-à-vis the available voice data. VMS **120** may monitor the PD status or condition in PD subjects, identify "on" and "off" conditions in the PD subjects and display, for the PD subject to see, a message or an alert, and/or send the message or alert to a physician using computer/server **134.**

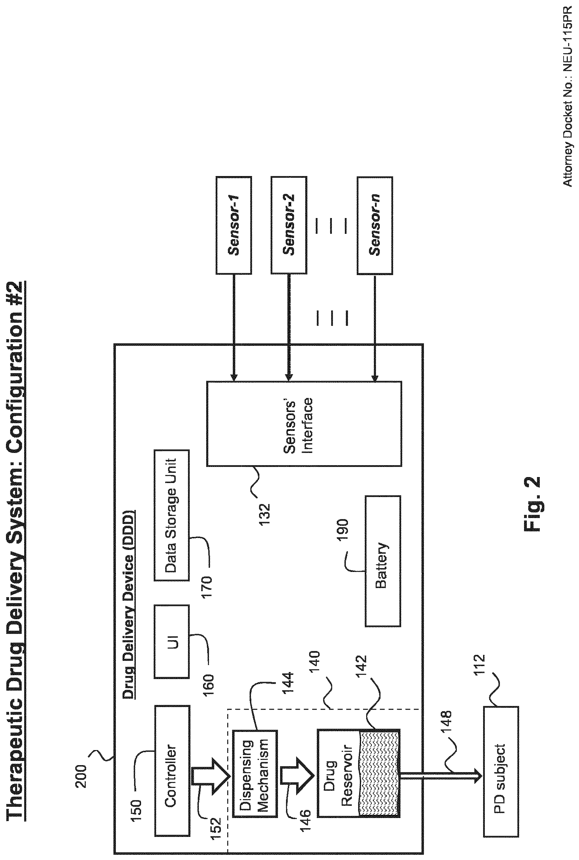

Fig. 2 shows another configuration of a drug delivery device (DDD) **200** for treating a condition associated with a Parkinson's disease (PD) subject, for example for preventing or delaying the onset of "off' periods, and/or for timewise shortening the duration of "off' periods, and/or for ameliorating HD symptom(s) or other symptom(s), according to an example embodiment. In Figs. 1 and 2 like reference numbers refer to like components/elements. For example, controller **150** of Fig. 2 may function in the way described herein in connection with controller **150** of Fig. 1; drug delivery unit (DDU) **140** of Fig. 2 may function in the way described herein in connection with DDU **140** of Fig. 1, and so on. Drug delivery device (DDD) **200** is also configured to perform functions/tasks that are performed, in the configuration of Fig. 1, by voice monitoring system **120.**

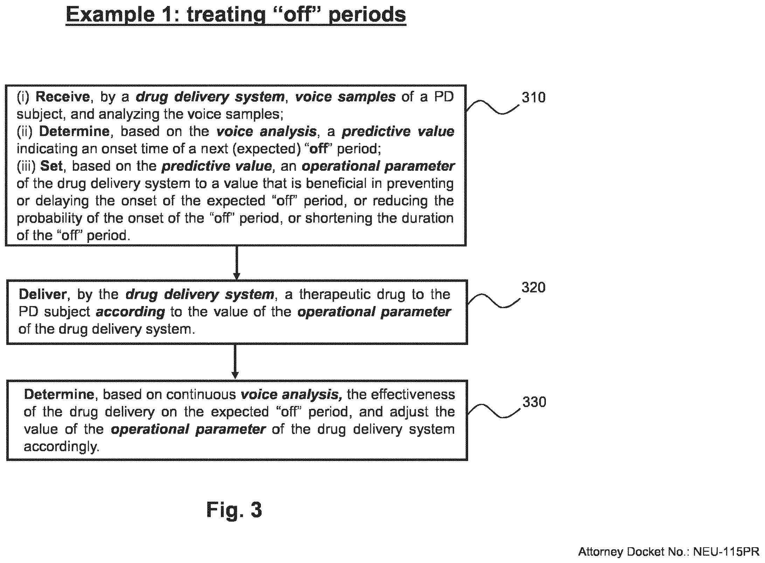

Fig. 3 shows a method of handling "off' periods in a PD subject according to an example embodiment. At step 310 a therapeutic drug delivery system (e.g., system 100 or 200) receives voice samples characterizing, for example, speech clarity of a PD subject, and analyzes the voice samples and determines, based on the voice analysis, a predictive value that indicates an onset time of the next (expected) "off' period. The predictive value may be determined, for example, in the following way: a change in a voice parameter is evaluated over time. If the value of the voice parameter changes by more than a preset threshold during a predetermined period, the parameter change indicates a corresponding change in the condition of the PD subject. For example, considering voice amplitude as a voice parameter, if the average voice amplitude decreases by more than 1 STD ('STD' - standard deviation) during a period of 10 minutes (for example), then a change from "on" period to "off' period is expected.

Based on the predictive value the therapeutic drug delivery system may set an operational parameter of the drug delivery system to a value that is beneficial in preventing or delaying the onset of the expected "off' period, or beneficial in reducing the probability of the occurrence of the onset of the "off' period, or beneficial in shortening the duration of the "off" period. At step 320 the therapeutic drug delivery system delivers a therapeutic drug to the PD subject according to the value of the operational parameter of the drug delivery system.

At step 330 the therapeutic drug delivery system may determine, based on continuous voice analysis, the effectiveness of the drug delivery on the expected "off" period (e.g., onset time of the expected "off" period and/or duration of the "off" period). The therapeutic drug delivery system may adjust the value of the operational parameter according to changes in the effectiveness of the drug delivery.

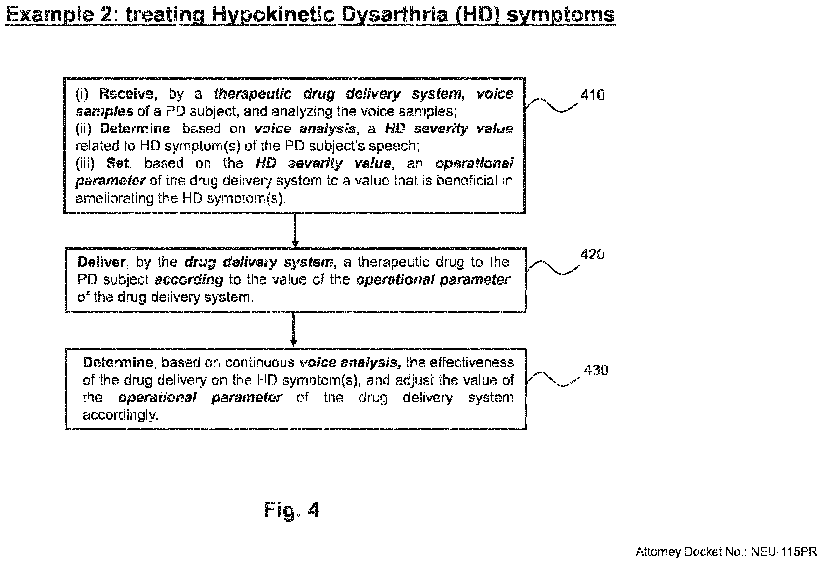

Fig. 4 shows a method of ameliorating HD symptom(s) in a PD subject according to an example embodiment. At step 410 a therapeutic drug delivery system (e.g., system **100** or **200**) receives voice samples characterizing HD symptom(s), analyzes the voice samples and determines, based on the voice analysis, a HD severity value that is related to HD symptom(s) in the PD subject. The HD severity value may be determined, for example, in the following way: for a voice parameter a parameter change is compared vis-à-vis the parameter historical values at a similar time of day. The severity value is determined by calculating the change in the parameter over a period of set days by more than the set threshold. For example, considering voice clarity as a parameter, if during a period of one month the average voice clarity over a period of a week was reduced by more than 1 STD, 2 STD, 3 STD, this may respectively indicate low severity level, medium severity level and high severity level. The therapeutic drug delivery system sets, based on the HD severity value, an operational parameter of the drug delivery system to a value that is beneficial in ameliorating the HD symptom(s).

At step 420 the therapeutic drug delivery system delivers a therapeutic drug to the PD subject according to the value set to the operational parameter of the drug delivery system. At step 430 the therapeutic drug delivery system determines, based on continuous voice analysis (based on a series of consecutive HD severity values), the effectiveness of the drug delivery on the HD symptom(s). The therapeutic drug delivery system may adjust the value of the operational parameter according to changes in the effectiveness of the drug delivery.

Having thus described exemplary embodiments of the invention, it will be apparent to those skilled in the art that modifications of the disclosed embodiments will be within the scope of the invention. Alternative embodiments may, accordingly, include functionally equivalent objects/articles. Features of certain embodiments may be used with other embodiments shown herein. The present disclosure is relevant to (e.g., it may be implemented by, used with or for) various types of Parkinson symptoms/disorders, non-motor symptoms, drug delivery pumps, therapeutic drugs, therapeutic drug dispensing devices, and the like. Hence the scope of the claims that follow is not limited by the disclosure herein.

Various aspects and embodiments may further be understood with reference to the following numbered clauses:
1. A method of evaluating a Parkinson disease (PD) condition in a PD subject, comprising:
   analyzing voice samples of the PD subject;
   determining voice characteristics of the PD subject based on the analysis of the voice samples; and
   evaluating a PD condition in the PD subject from the voice characteristics.
2. The method of clause 1, wherein the PD condition is selected from the group consisting of: (1) a future onset and duration of an "off" period, (2) a state of a Hypokinetic dysarthria (HD) symptom, (3) depression, (4) dementia, and (5) Alzheimer.
3. The method of clause 2, further comprising calculating a predictive value from voice samples, the predictive value predicting an expected onset of an "off" period and the duration of the expected "off" period.
4. The method of clause 1, 2 or 3, further comprising calculating a PD severity value from voice samples, the PD severity value indicating a severity of a PD condition.
5. The method of clause 4, wherein the PD severity value is a HD severity value, the HD severity value indicating a severity of a HD symptom.
6. The method of any one of clauses 1 to 5, wherein the voice samples comprise historical voice samples, current voice samples and reference voice samples representing spoken word(s), phrase(s), sentence(s), consonant(s) and/or vowel(s).
7. The method of clause 6, wherein the reference voice samples represent the PD subject own spoken word(s), phrase(s), sentence(s), consonant(s) and/or vowel(s).
8. The method of clause 6 or 7, further comprising determining historical voice characteristics from the historical voice samples and correlating between the historical voice characteristics and historical onset and duration of one or more PD "off' periods to determine an onset time and a duration of a future "off" period.
9. The method of any one of clauses 6 to 8, further comprising determining historical voice characteristics from historical voice samples and correlating between the historical voice characteristics and historical Hypokinetic dysarthria (HD) symptom or other PD symptom to evaluate a progression of the HD symptom or another PD symptom.
10. The method of any one of clauses 1 to 9, wherein the voice samples are obtained during random phone calls of the PD subject, or during prescheduled phone calls of the PD subject.
11. The method of clause 10, wherein the timing of a phone call is scheduled to an expected PD "off" period or "on" period.
12. The method of any one of clauses 1 to 11, further comprising using voice and sound sampling during sleep to measure breathing sounds, snoring sounds and noises made by bed movement, and using the breathing sounds, snoring sounds and bed movement noises in combination with voice analysis to optimize or enhance detection of a speech problem or another PD condition of the PD subject.
13. The method according to clause 6 or any one of clauses 7 to 12 depending from clause 6, wherein evaluation of the progression of the PD condition comprises providing historical voice samples to a machine learning algorithm as a training set, wherein the historical voice samples represent historical "on" and "off periods, or historical HD symptom(s), or other historical PD symptom(s).
14. The method of any one of clauses 1 to 13, wherein evaluation of the progression of the PD condition is performed by detecting changes in a voice parameter or characteristic.
15. The method of any one of clauses 1 to 14, further comprising adjusting, based on the evaluation of the PD condition, drug delivery to the PD subject to prevent or delay an expected "off period and/or to shorten the duration of the "off period and/or to mitigate other PD symptoms.
16. The method of any one of clauses 1 to 15, further comprising adjusting drug delivery to the PD subject to ameliorate a PD symptom.
17. A voice monitoring system (VMS) for evaluating a Parkinson disease (PD) condition in a PD subject, comprising:
   a controller configured to,
   (i) analyze voice samples of the PD subject that are stored in a data storage unit;
   (ii) determine voice characteristics of the PD subject based on the analysis of the voice samples; and
   (iii) evaluate a status of a PD condition from the voice characteristics.
18. The system of clause 17, wherein the PD condition is selected from the group consisting of: (1) a future onset and duration of "on" and "off periods, (2) a state of a Hypokinetic dysarthria (HD) symptom, (3) depression, (4) dementia, and (5) Alzheimer.
19. The system of clause 18, wherein the controller is configured to calculate a predictive value, the predictive value predicting a future onset and duration of an "off period.
20. The system of any one of clauses 17 to 19, wherein the controller is configured to calculate a PD severity value from voice samples, the PD severity value indicating a severity of a PD condition.
21. The system of clause 20, wherein the PD severity value is a HD severity value, the HD severity value indicating a severity of a HD symptom.
22. The system of any one of clauses 17 to 21, wherein the voice samples comprise historical voice samples, current voice sample, and reference voice samples representing spoken word(s), phrase(s), sentence(s), consonant(s) and/or vowel(s).
23. The system of clause 22, wherein the reference voice samples represent the PD subject own spoken word(s), phrase(s), sentence(s), consonant(s) and/or vowel(s).
24. The system of clause 22 or 23, wherein the controller is configured to determine historical voice characteristics from historical voice samples and to use correlation between the historical voice characteristics and historical onset of one or more PD "off" periods to determine an onset time and duration of a future "off' period.
25. The system of any one of clauses 22 to 24, wherein controller is configured to determine historical voice characteristics from historical voice samples and to use correlation between the historical voice characteristics and historical Hypokinetic dysarthria (HD) symptoms to evaluate a progression of the HD symptom or other PD symptom.
26. The system of any one of clauses 17 to 25, wherein the controller is configured to sample voice during random phone calls of the PD subject, or during prescheduled phone calls of the PD subject.
27. The system of clause 26, wherein the controller is configured to schedule a phone call to an expected PD "off" period or "on" period.
28. The system according to clause 24 or any one of clauses 25 to 27 depending from clause 24, wherein the controller is configured to train a machine learning algorithm by providing historical voice samples to the machine learning algorithm as a training set, wherein the historical voice samples represent historical "on" and "off" periods, or historical HD symptom(s) or other historical PD symptom(s).
29. The system of any one of clauses 17 to 28, wherein the controller is configured to evaluate a PD condition or a progression of the PD condition by detecting changes in a voice parameter.
30. The system of any one of clauses 17 to 29, wherein the controller is configured to adjust a treatment or to recommend an adjustment to a drug delivery parameter to prevent or delay the onset of an expected "off" period and/or to shorten the duration of the "off" period.
31. The system of any one of clauses 17 to 30, wherein the controller is configured to adjust treatment or to recommend an adjustment to a drug delivery parameter to ameliorate a Hypokinetic dysarthria (HD) symptom or another PD symptom.

## Claims

1. A voice monitoring system (VMS) for evaluating a Parkinson disease (PD) condition in a PD subject, comprising:
a controller configured to,
(i) analyze voice samples of the PD subject that are stored in a data storage unit;
(ii) determine voice characteristics of the PD subject based on the analysis of the voice samples; and
(iii) evaluate a status of a PD condition from the voice characteristics.

2. The system of claim 1, wherein the PD condition is selected from the group consisting of: (1) a future onset and duration of "on" and "off" periods, (2) a state of a Hypokinetic dysarthria (HD) symptom, (3) depression, (4) dementia, and (5) Alzheimer.

3. The system of claim 2, wherein the controller is configured to calculate a predictive value, the predictive value predicting a future onset and duration of an "off" period.

4. The system of claim 1, 2 or 3, wherein the controller is configured to calculate a PD severity value from voice samples, the PD severity value indicating a severity of a PD condition; optionally wherein the PD severity value is a HD severity value, the HD severity value indicating a severity of a HD symptom.

5. The system of any preceding claim, wherein the voice samples comprise historical voice samples, current voice sample, and reference voice samples representing spoken word(s), phrase(s), sentence(s), consonant(s) and/or vowel(s); optionally wherein the reference voice samples represent the PD subject own spoken word(s), phrase(s), sentence(s), consonant(s) and/or vowel(s).

6. The system of claim 5, wherein the controller is configured to determine historical voice characteristics from historical voice samples and either to use:
i) correlation between the historical voice characteristics and historical onset of one or more PD "off" periods to determine an onset time and duration of a future "off" period; or
ii) correlation between the historical voice characteristics and historical Hypokinetic dysarthria (HD) symptoms to evaluate a progression of the HD symptom or other PD symptom.

7. The system of any preceding claim, wherein the controller is configured to sample voice during random phone calls of the PD subject, or during prescheduled phone calls of the PD subject; optionally wherein the controller is configured to schedule a phone call to an expected PD "off" period or "on" period.

8. The system according to claim 6, wherein the controller is configured to train a machine learning algorithm by providing historical voice samples to the machine learning algorithm as a training set, wherein the historical voice samples represent historical "on" and "off' periods, or historical HD symptom(s) or other historical PD symptom(s).

9. The system of any preceding claim, wherein the controller is configured to evaluate a PD condition or a progression of the PD condition by detecting changes in a voice parameter.

10. The system of claim 2 or any one of claims 3 to 9 depending from claim 2, wherein the controller is configured to adjust a treatment or to recommend an adjustment to a drug delivery parameter to prevent or delay the onset of an expected "off" period and/or to shorten the duration of the "off" period; and/or wherein the controller is configured to adjust treatment or to recommend an adjustment to a drug delivery parameter to ameliorate a Hypokinetic dysarthria (HD) symptom or another PD symptom.

11. A method of evaluating a Parkinson disease (PD) condition in a PD subject, comprising:
analyzing voice samples of the PD subject;
determining voice characteristics of the PD subject based on the analysis of the voice samples; and
evaluating a PD condition in the PD subject from the voice characteristics.

12. The method of claim 11, wherein the PD condition is selected from the group consisting of: (1) a future onset and duration of an "off" period, (2) a state of a Hypokinetic dysarthria (HD) symptom, (3) depression, (4) dementia, and (5) Alzheimer.

13. The method of claim 12, further comprising calculating a predictive value from voice samples, the predictive value predicting an expected onset of an "off" period and the duration of the expected "off" period.

14. The method of any one of claims 11 to 13, further comprising calculating a PD severity value from voice samples, the PD severity value indicating a severity of a PD condition; optionally wherein the PD severity value is a HD severity value, the HD severity value indicating a severity of a HD symptom.

15. The method of any one of claims 11 to 14, wherein the voice samples comprise historical voice samples, current voice samples and reference voice samples representing spoken word(s), phrase(s), sentence(s), consonant(s) and/or vowel(s); optionally wherein the reference voice samples represent the PD subject own spoken word(s), phrase(s), sentence(s), consonant(s) and/or vowel(s).

16. The method of claim 15, further comprising determining historical voice characteristics from the historical voice samples and either:
i) correlating between the historical voice characteristics and historical onset and duration of one or more PD "off' periods to determine an onset time and a duration of a future "off" period; or
ii) correlating between the historical voice characteristics and historical Hypokinetic dysarthria (HD) symptom or other PD symptom to evaluate a progression of the HD symptom or another PD symptom.

17. The method according to claim 16, wherein evaluation of the progression of the PD condition comprises providing historical voice samples to a machine learning algorithm as a training set, wherein the historical voice samples represent historical "on" and "off" periods, or historical HD symptom(s), or other historical PD symptom(s).

18. The method of claim 16 or 17, wherein evaluation of the progression of the PD condition is performed by detecting changes in a voice parameter or characteristic.
